# EUROPÄISCHE PATENTANMELDUNG

(11) **EP 1 121 903 A2**
(43) Veröffentlichungstag der Anmeldung: **08.08.2001**
(21) Anmeldenummer: 01250026.0
(22) Anmeldetag: 25.01.2001
(51) Int. Cl.: A61B 17/70, A61B 17/80

(54) **Osteosynthese-Platte**

(30) Priorität: 25.01.2000 DE 10003968
(71) Anmelder: Biomet Merck Deutschland GmbH, 14167 Berlin (DE)
(72) Erfinder: Calisse, Jorge, Dr., 10785 Berlin (DE); Klas, Norbert, Dr., 64625 Bensheim (DE)
(74) Vertreter: Gross, Felix, Dr.

(57) **Zusammenfassung**

Die Erfindung betrifft eine zur Stabilisierung der relativen Stellung von Wirbelknochen, insbesondere von Wirbelknochen (2, 3) im Bereich der menschlichen Wirbelsäule, dienende Osteosynthese-Platte (1, 10, 20, 30, 40, 50), welche in Längs- und Querrichtung jeweils eine konkave Wölbung (4.1, 4.2, 14.2, 24.1, 24.2, 31.2, 42.2, 52.2) und in ihren mit den Wirbelknochen in Kontakt zu bringenden Bereichen (5, 15, 25, 31.3, 42.3, 52.3) zum Einbringen von Schraubmitteln angeordneten Bohrungen (8,18.28,38,48,58) aufweist, dadurch gekennzeichnet, daß an den Kontaktbereichen der Platte jeweils eine Ausformung vorgesehen ist, die eine bezüglich der Wölbungen (4.1, 4.2, 14.2, 24.1, 24.2, 31.2, 42.2, 52.2) entgegengesetzt gerichtete, konvexe Gegenwölbung (6, 16, 26, 36, 46, 56) bildet.

## Beschreibung

Die Erfindung betrifft eine Osteosynthese-Platte zum Stabilisieren der relativen Position von Wirbelkörpern, insbesondere für eine Anwendung im Bereich der Wirbelsäule des Menschen. Die Osteosynthese-Platte weist in Längs- und Querrichtung zur Stabilisierung der relativen Stellung von Wirbelkörpern eine konkave Wölbung und in ihren Endbereichen zum Einbringen von Schraubmitteln angeordnete Bohrungen auf.

Aus der internationalen Patentanmeldung WO 94/17744 ist eine Osteosynthese-Platte zum Fixieren von Wirbelknochen unter Verwendung von Knochenschrauben bekannt. Diese Platte weist sowohl in Richtung ihrer Längserstreckung als auch in Richtung ihrer Quererstreckung eine konvexe Wölbung nach distal auf. An den sich einander gegenüberliegenden Enden der Osteosynthese-Platte sind jeweils paarweise Bohrungen vorgesehen, welche zur Aufnahme von Knochenschrauben zur Verschraubung der Platte mit den betreffenden Wirbelkörpern dienen.

Bei der bekannten Lösung besteht jedoch in nachteiliger Weise die Gefahr, daß in Abhängigkeit von - patientenspezifisch bedingt - anatomischen Unterschieden der Wirbelkörperform die medizinisch optimale Plazierung der Osteosynthese-Platte erheblich erschwert wird, wenn nicht alle zur Verschraubung vorgesehenen Bereiche vollflächig an der Knochenoberfläche des Wirbelkörpers anliegen und es dadurch zu unerwünschten und in der Größe nicht genau bestimmbaren Verspannungen im Wirbelbereich kommen kann, wenn die Fixierung der Osteosynthese-Platte an den Wirbelkörpern vorgenommen wird.

Ausgehends von den Mängeln des Standes der Technik liegt der Erfindung liegt deshalb die Aufgabe zugrunde, eine Osteosynthese-Platte der vorstehend genannten Gattung anzugeben, welche sich besonders einfach im Bereich der menschlichen Wirbelsäule zwecks Stabilisierung benachbarter Wirbelkörper positionieren und fixieren läßt.

Die Aufgabe wird durch eine Osteosynthese-Platte der eingangs genannten Art gelöst, welche an ihren mit der Knochenoberfläche der Wirbelkörper in Kontakt stehenden Bereichen jeweils eine Ausformung aufweist, die eine bezüglich der Wölbungen entgegengesetzt gerichtete, konvexe Gegenwölbung bildet. Diese Kontaktbereiche befinden sich im wesentlichen an den Endabschnitten der erfindungsgemäßen Osteosynthese-Platte.

Die Erfindung schließt die Erkenntnis ein, daß besondere Vorteile bei einer ortsfesten Fixierung einer Osteosynthese-Platte zwecks Stabilisierung zweier benachbarter Wirbelkörper erreichbar ist, wenn die Bereiche der Platte, an welchen die Verbindung mit den betreffenden Wirbelkörper hergestellt werden soll, derart ausgebildet sind, daß - von der äußeren Form der Wirbelkörper im wesentlichen unabhängig - stets eine genügend große Auflagefläche zwischen der Knochenmasse des Wirbelkörpers und der Osteosynthese-Platte an den Stellen vorhanden ist, wo die zum Fixieren dieser Platte verwendeten Knochenschrauben plaziert gesetzt werden sollen.

Erfindungsgemäß ist bei der zur Stabilisierung der relativen Stellung von Wirbelkörper, insbesondere von Wirbelkörper im Bereich der menschlichen Wirbelsäule einsetzbaren Ostheosynthese-Platte, welche in ihrem Mittelbereich jeweils eine konkaven Wölbung in Längs- und Quererstreckung und in ihren Kontaktbereichen zum Einbringen von Schraubmitteln angeordnete Bohrungen aufweist, an diesen Endbereichen der Platte jeweils eine bezüglich der konkaven Wölbungen im Mittelbereich eine als konvexe Gegenwölbung ausgebildete Ausformung vorgesehen. Die Größe dieser Wölbungen, insbesondere der Gegenwölbung, ist derart bemessen, daß bei an den Wirbelkörper fixierter Platte zwischen Wirbelkörpern und einem Mittelbereich der Platte ein Freiraum verbleibt.

Durch die an der Ostesynthese-Platte erfindungsgemäß vorgesehene konvexe Gegenwölbung ist gesichert, daß die Platte auf der Außenseite der zu stabilisierenden Wirbelkörper auf einfache Weise so plaziert werden kann, daß stets alle Endbereiche der Platte im wesentlichen flächig an der Knochenoberfläche der Wirbelkörper aufliegen. Die Knochenschrauben zum Fixieren der Platte an den Wirbelkörpern können dadurch in vorteilhafter Weise so gesetzt werden, daß keine unerwünschten Verspannungen auftreten.

Durch den nach Fixieren einer erfindungsgemäßen Osteosynthese-Platte zwischen Platte und Wirbelkörpern vorhandenen Freiraum wird gleichzeitig in vorteilhafter Weise erreicht, daß sich die Traumatisierung der an den Foramina-Punkten der Wirbelsäule austretenden Nervenfasern nach dem chirurgischen Eingriff verringert, was einerseits zu einer Reduzierung der Belastung des Patienten führt und andererseits auch den Heilungsprozeß beschleunigt.

Entsprechend der bevorzugten Ausführungsform der Erfindung weisen die Wölbungen die Form einer Kugelkappe auf. Dabei ist es für eine günstige Kräfteverteilung sowie für ein bequemes Positionieren der Osteosynthese-Platte an den betreffenden Wirbelkörpern günstig, wenn die an den Kontaktbereichen der Platte vorgesehenen konvexen Gegenwölbungen einen Radius mit einem Wert aufweisen, welcher im wesentlichen dem Wert des Wölbungsradius in der Längsrichtung der Osteosynthese-Platte entspricht.

Überraschender Weise wurde festgestellt, daß entsprechend einer günstigen Weiterbildung der Erfindung eine schmetterlingsförmig ausgebildete Osteosynthese-Platte in der praktischen Anwendung besonders vorteilhaft ist.

Für eine technologisch wenig aufwendige Fertigung der erfindungsgemäßen Osteosynthese-Platte ist es vorteilhaft, wenn die Plattendicke einen im wesentlichen konstanten Wert aufweist. Dadurch kann die Platte mittels formgebendem Stanzen aus einem Flachmaterial, beispielsweise aus rostfreiem Stahlblech, hergestellt werden. Die erforderliche Festigkeit wird sowohl durch die Wahl der Materialkennwerte des Werkstoffs als auch durch die erfindungsgemäße Formgebung gesichert.

Nach der bevorzugten Ausführungsform der Erfindung sind an der konvexen Seite der gewölbten Endbereiche der osteosynthetischen Platte Lagefixierungsmittel mit geringer Höhe vorgesehen. Diese Lagefixierungsmittel erstrecken sich an der konvexen Seite der Ausformung nach außen. Sie sind entsprechend einer Variante der Erfindung dornen- oder noppenförmig ausgebildet und weisen bevorzugt im wesentlichen die Form eines Pyramiden- oder Kegelstumpfes auf. Die Achse der Zähne oder Noppen erstreckt sich in günstiger Weise parallel zur Achse der für die Kochenschrauben vorgesehenen Bohrungen der Osteosynthese-Platte, um zu erreichen, daß die Lagefixierungsmittel beim Anziehen der Knochenschrauben optimal mit der Knochenoberfläche der Wirbelkörper in Wirkungseingriff gelangen. Durch Anordnung derartig geformter Lagefixierungsmittel ist trotz der in Bezug auf die Plattengröße relativ geringen Auflagefläche ein fester Sitz der Osteosynthese-Platte an den zu stabilisierenden Wirbelkörpern gesichert.

Entsprechend einer vorteilhaften Variante der Erfindung sind die an den Kontaktbereichen der Osteosynthese-Platte angeordneten Bohrungen mit einer Senkung, bevorzugt eine sphärische Senkung, versehen. Derartige Kugelsenkungen weisen den besonderen Vorteil auf, daß sich das Setzen der Knochenschrauben zum Fixieren der Osteosynthese-Platte vereinfacht, da ein fester Sitz des Schraubenkopfes im wesentlichen unabhängig vom Einschraubwinkel in bezug auf die Achse der Bohrung erreichbar ist. Um ein unbeabsichtigtes Herausfallen der Knochenschrauben aus der Osteosynthese-Platte zu verhindern, ist erfindungsgemäß in den an den Endbereichen vorhandenen Bohrungen mindestens ein Sicherungsmittel vorgesehen. Dieses Sicherungsmittel ist bevorzugt als Haltenase ausgebildet, welche sich radial in den Innenraum der jeweiligen Bohrung erstreckt und ein Einbringen bzw. Entfernen der Knochenschraube in bzw. aus der Bohrung nur durch Drehen derselben möglich ist.

Entsprechend einer anderen Ausführungsform der Erfindung wird die Osteosynthese-Platte durch Gießen bzw. Schmieden hergestellt, wobei die mit einer konvexe Gegenwölbung versehenen Kontaktbereiche der Platte eine größere Materialdicke aufweisen als der Mittelbereich der ansonsten im wesentlichen unveränderten Platte. Eine derartige Form der Osteosynthese-Platte ist nach einer Variante der Erfindung auch auf einfache Weise durch eine spangebende Bearbeitung aus einem metallischen Halbzeug kostengünstig herstellbar.

Nach einer anderen vorteilhaften Ausführungsform der Erfindung ist U-förmig ausgebildet oder weist die Osteosynthese-Platte die Form eines Doppel-T auf, wobei die vorstehend beschriebenen Wölbungsverhältnisse, d.h. Wölbung und Gegenwölbung im Mittenbereich bzw. an den Kontaktbereichen, im wesentlichen beibehalten werden.

Entsprechend einer zusätzlichen Ausführungsform der Erfindung ist die Osteosynthese-Platte aus modulartigen Einzelteilen zusammensetzbar ausgebildet. Als Einzelteile sind in vorteilhafter Weise ein in Längserstreckung wellenförmiger Träger und mehrere, sich im wesentlichen quer zu dem Träger erstreckende Querstege vorgesehen, welche jeweils in den benachbarten Wellenbergen des Trägers, bevorzugt durch Schraubmittel, lösbar befestigt sind. Die Querstege weisen im Mittenbereich und an den Enden jeweils eine Wölbung bzw. eine Gegenwölbung sowie Lagefixierungsmittel entsprechend den vorstehend beschriebenen Ausführungsformen der erfindungsgemäßen Osteosynthese-Platte auf.

Durch ein derartiges Modulsystem ist beispielsweise eine Osteosynthese-Platte in Form eines doppelten Doppel-T auf einfache Weise bildbar, wobei durch Einstellmittel, beispielsweise durch in dem Träger oder in den Querstegen vorgesehene Länglöcher, im begrenzten Umfang eine Veränderung des Abstands zwischen benachbarten Querstegen und damit eine Anpassung der Platte an die patientenspezifische Anatomie der Wirbelsäule erreichbar ist, um eine spezielle Therapie durchführen zu können.

Entsprechend einer anderen Variante der Erfindung ist die den Wirbelknochen zugewandte Oberfläche der Osteosynthese-Platte mit einer Rauhigkeit versehen. Diese Rauhigkeit, welche - ebenso wie die vorstehend beschriebenen Lagefixierungsmittel - der positionierten Osteosynthese-Platte am Wirbelknochen einen zusätzliche Halt gibt, wird durch Sandstrahlen oder durch eine spezielle Plasmabeschichtung der Plattenoberfläche erreicht.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt.

Es zeigen:
- Figur 1 eine bevorzugte Ausführungsform der Erfindung,
- Figur 2 die in Figur 1 gezeigte Ausführungsform der Erfindung in Teilschnittansicht von der Seite,
- Figur 3 die in Figur 2 dargestellte Ausführungsform der Erfindung in Draufsicht,
- Figur 4 die Darstellung der Ansicht eines Schnittes längs der Linie A...A in Figur 3,
- Figur 5 eine günstige Weiterbildung der in den Figuren 2, 3 und 4 gezeigten Erfindung in Schnittdarstellung,
- Figuren 6 bis 8 eine andere günstige Ausführungsform der Erfindung in seitlicher Teilschnittansicht, in Draufsicht bzw. in Schnittdarstellung.
- Figur 9 eine Variante der Erfindung in Draufsicht,
- Figur 10 die Ansicht des Schnittes längs der Linie C...C gemäß Figur 9,

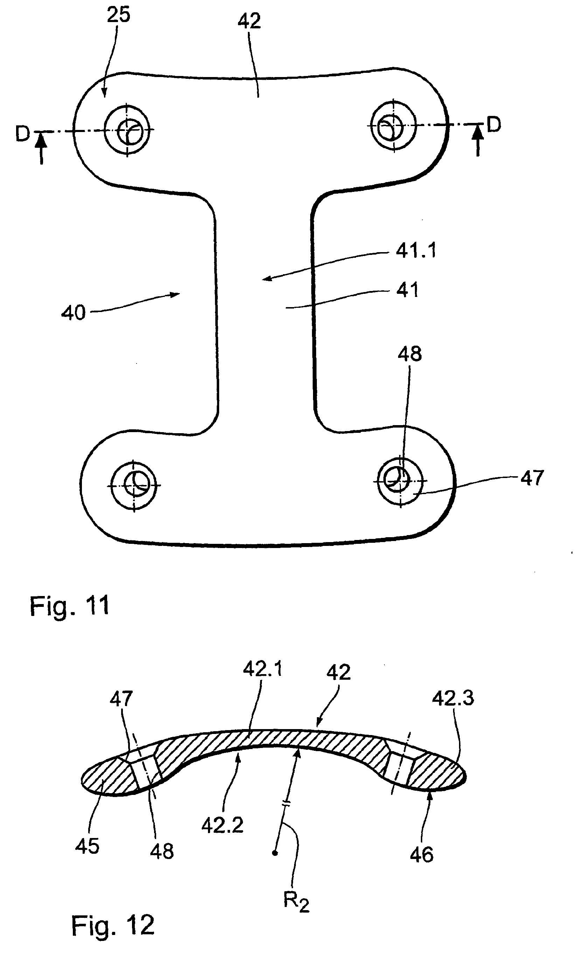

- Figur 11 eine weitere Variante der Erfindung in Draufsicht,
- Figur 12 die Ansicht des Schnittes längs der Linie D...D gemäß Figur 11,
- Figur 13 eine zusätzliche Ausführungsform der Erfindung in Draufsicht sowie
- Figuren 14 und 15 die Darstellung der Ansicht eines Schnittes längs der Linien E...E bzw. F...F in Figur 13.

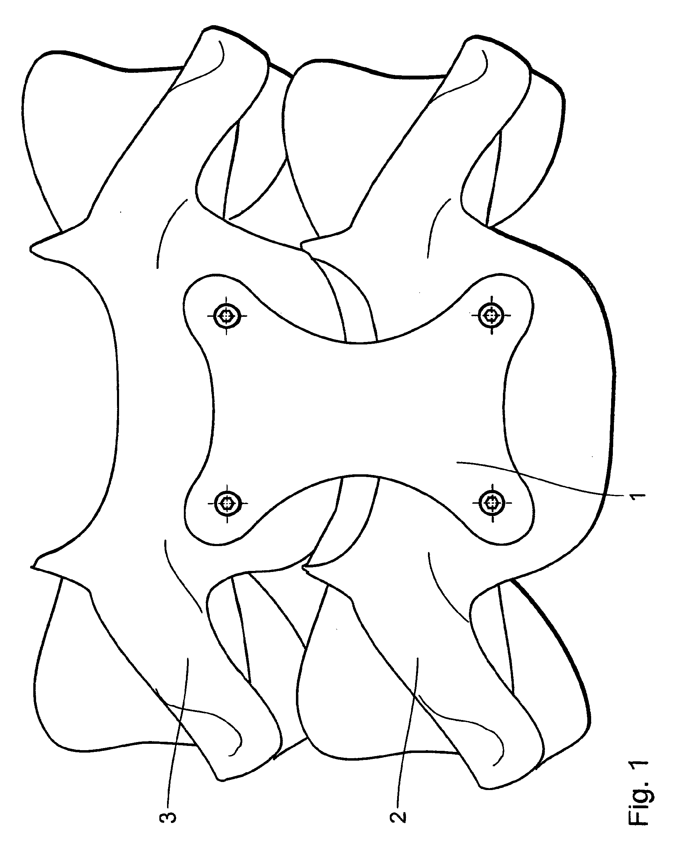

Die in den Figuren 1, 2, 3 und 4 gezeigte Osteosynthese-Platte 1 dient zur mechanischen Stabilisierung benachbarter Wirbelkörper 2, 3.

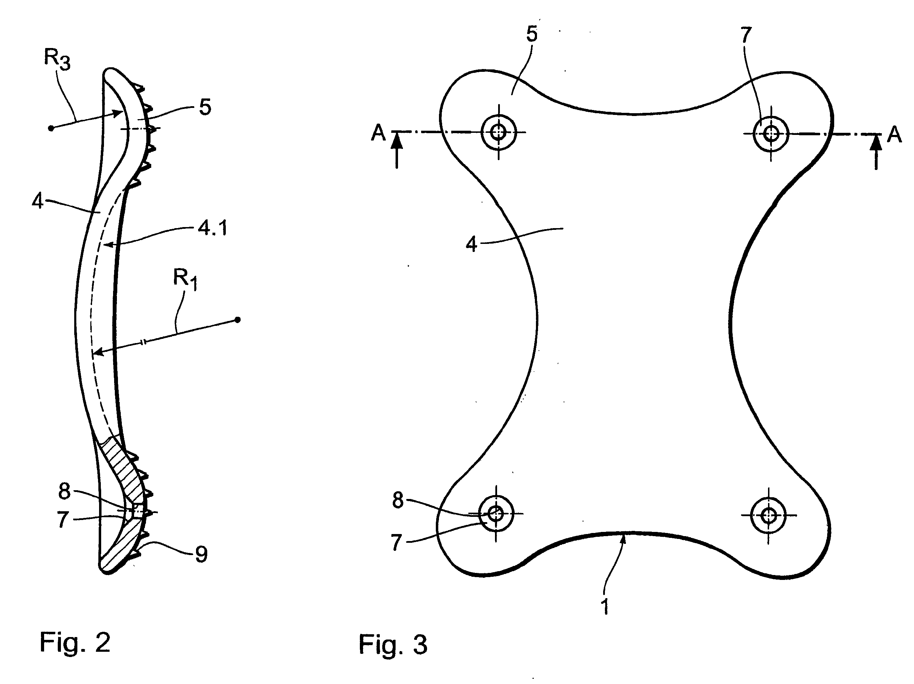

Die Platte 1 ist - wie in Figur 1 und 3 dargestellt - schmetterlingsförmig ausgebildet und weist ein Mittelbereich 4 auf, welcher sowohl in Längsrichtung als auch in Querrichtung der Platte 1 gewölbt ist. Die entsprechenden Wölbungsradien sind mit R₁ und R₂ bezeichnet. Die Wölbungen sind in der gleichen Richtung konkav ausgebildet und mit 4.1 bzw. 4.2 bezeichnet.

Die vier Kontaktbereiche 5, in welche der Mittelbereich 4 der Osteosynthese-Platte 1 ausläuft, sind jeweils unter Bildung einer in Bezug auf die beiden Wölbungen 4.1 und 4.2 entgegengesetzt gerichteten konvexen Wölbung 6 ausgeformt. Der Radius dieser Gegenwölbung 6 ist mit R₃ bezeichnet. Die Endbereiche 5 liegen bei der Stabilisierung der Wirbelknochen 2, 3 auf der Knochenoberfläche und weisen zwecks Verschraubung jeweils eine mit einer Konussenkung 7 versehene Bohrung 8 auf.

An der den zu stabilisierenden Wirbelkörper 2, 3 zugewandten konvexen Seite der die Gegenwölbung 6 bildenden Ausformung sind als dornenförmiger Pyramidenstumpf 9 ausgebildete Materialerhebungen geringer Höhe vorgesehen, welche sich an der konvexen Wölbung nach außen erstrecken. Sie sind gleichmäßig auf der Wölbungsoberfläche verteilt angeordnet, wobei sich die Achse der jeweiligen Materialerhebung in Richtung der Längsachse der zur Aufnahme der Knchenschrauben vorgesehenen Bohrungen erstreckt.

Die medizinisch erforderliche Position der an den Wirbelkörper zu fixierenden Osteosynthese-Platte kann wegen der erfindungsgemäßen Plattenform bequem gewählt werden, ohne daß die Gefahr besteht, daß einer der Kontaktbereiche der Platte nicht zur Anlage auf der Knochenoberfläche kommt.

Die Osteosynthese-Platte 1 weist eine konstante Materialstärke auf. Sie kann auf einfache Weise durch formgebendes Stanzen aus einem Blech hergestellt werden, wobei die geforderte Festigkeit durch die Wahl des Werkstoffs, bevorzugt rostfreier Stahl, Titan oder einer Titanlegierung und die durch die Wölbungen 4.1,4.2 und 6 bestimmte Formgebung problemlos erreicht wird.

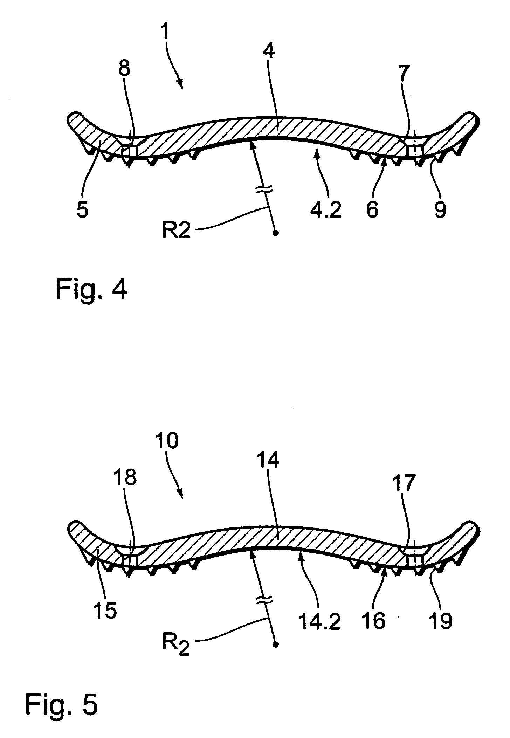

Bei der in Figur 5 gezeigten Ausführungsform der Erfindung weist die Osteosynthese-Platte 10 Bohrungen 18 zum Setzen von Knochenschrauben auf, welche mit einer sphärischen Senkung 17 versehen sind. Derartige Senkungen haben den Vorteil, daß das Fixieren der Osteosynthese-Platte mittels Knochenschrauben vereinfacht wird, da ein fester Sitz der Knochenschrauben auch dann gesichert ist, wenn die Schraubenachse und die Achse der Bohrung beim Einschrauben nicht übereinstimmen. Die Unterteilung der Osteosynthese-Platte 10 in Mittenbereich 14 und Endbereiche 15 erfolgt in gleicher Weise die vorstehend beschrieben. Wölbung und Gegenwölbung sind mit 14 und 16 bezeichnet. Die Fixierungsmittel sind noppenförmig ausgebildet und tragen die Positionsziffer 19.

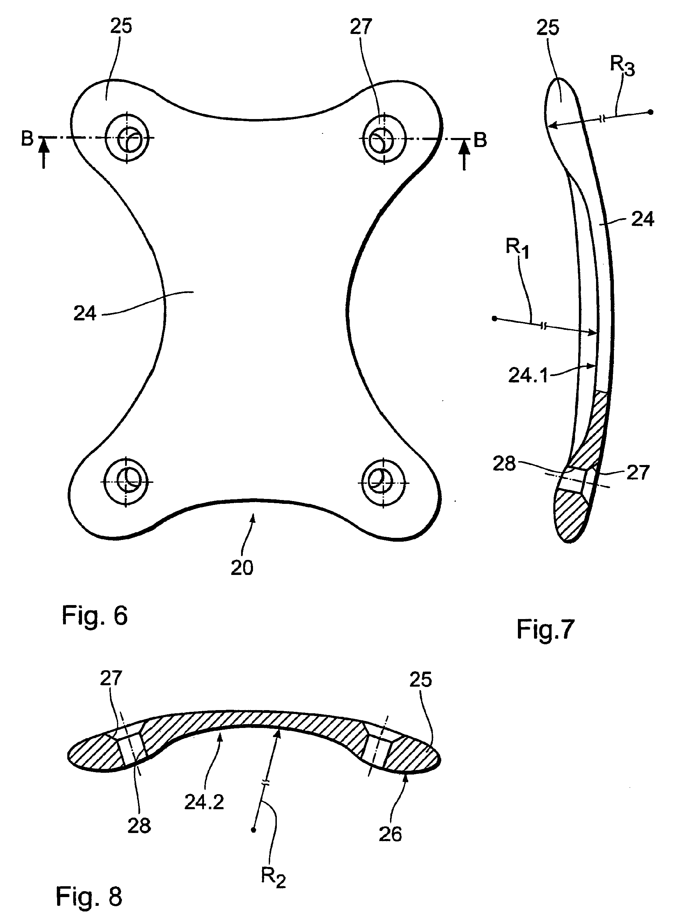

Figuren 6 bis 8 zeigen eine aus Titan gegossene Ausführungsform der Osteosynthese-Platte 20, welche sich im Vergleich mit den vorstehend beschriebenen Ausführungsformen der Osteosynthese-Platten 1 und 10 durch die im Verhältnis zu dem Mittelbereich 24 verdickten Endbereiche 25 unterscheidet. Die relativ große Materialstärke der an den Plattenenden befindlichen Kontaktbereichen gestattet eine höhere mechanische Belastung und den Einsatz von Knochenschrauben mit größerem Durchmesser beim Fixieren der Osteosynthese-Platte 20. Die in der als Seitenansicht gezeigte Teilschnittdarstellung (Figur 7) und in der Ansicht des Schnittes längs der Linie B...B gemäß Figur 6 (Figur 8) dargestellten Wölbungen sind mit 24.1, 24.2 bzw. 26 bezeichnet. Die mit einer Senkung 27 versehenen Bohrungen 28 in den Kontaktbereichen sind entsprechend Figur 3 angeordnet.

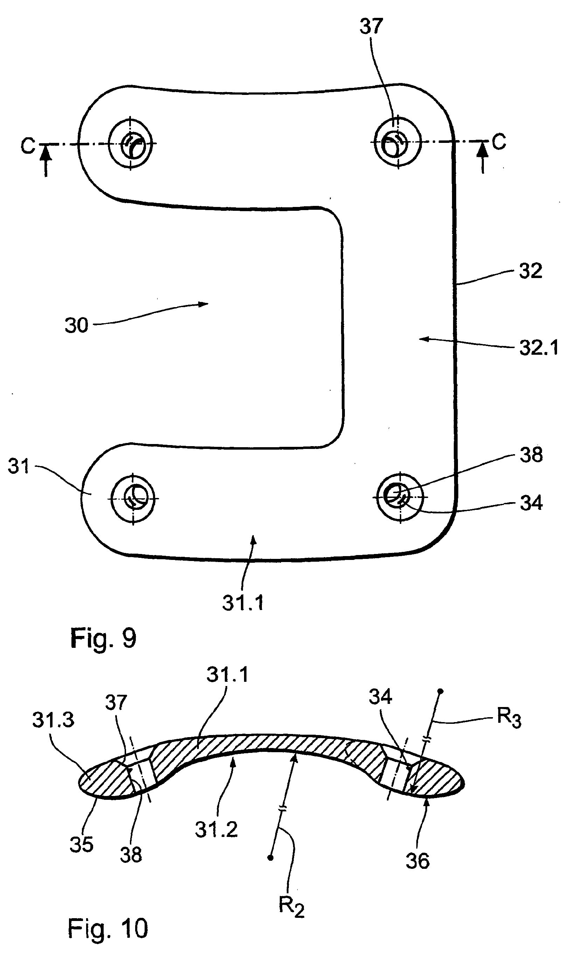

In den Figuren 9 und 10 ist eine U-förmige Osteosynthese-Platte 30 dargestellt, bei welcher sowohl die Schenkel 31 als auch der Bodenteil 32 des U einen Mittenbereich 31.1, 32.1 mit einer konkaven Wölbung 31.2 (Radius R₂) aufweisen. Gleichermaßen sind die nicht gesondert bezeichneten Kontaktbereiche von Schenkel 31 und Bodenteil 32 des U jeweils mit einer Gegenwölbung 36 (Radius R₃) versehen.

Zum Fixieren der Osteosyntese-Platte 30 an den Wirbelknochen durch Knochenschrauben sind an den Endbereichen von Schenkel und Bodenteil des U jeweils mit einer Senkung 37 versehene Bohrungen 38 angeordnet. Eine an der Plattenunterseite vorgesehene Plasmabeschichtung 35 unterstützt durch ihre hohe Rauhigkeit den festen Sitz der Platte 30.

In jeder Bohrung 38 ist eine Haltenase 34 angeordnet, welche sich radial in den Innenraum der Bohrung 38 erstreckt. Die Abmessungen der Haltenase 34 sind derart gewählt, daß sie bequem in den zwischen den Gewindegängen der (nicht dargestellten) Knochenschrauben Platz findet und die jeweilige Halteschraube durch Drehen in die Bohrung eingebracht bzw. aus ihr entfernt werden kann. Bei eingeschraubter Knochenschraube befindet sich die Haltenase 34 am Schraubenhals oberhalb des Gewindebereichs der Schraube.

Die in den Figuren 1 1 und 12 dargestellte Osteosynthese-Platte 40 ist als Doppel-T ausgebildet.

Sowohl der Träger 41 als auch die beiden Querstege 42 weisen in ihrem mittleren Bereich 41.2,42.1 eine konkave Wölbung 42.2 (Radius R₂). Die konkave Wölbung 42.2 geht an den freien Enden der Querstege 42 in eine konvexe Gegenwölbung 46 über. Die Unterseite 45 der Osteosynthese-Platte 40 ist durch Sandstrahlen aufgerauht, wodurch das Fixieren der Osteosynthese-Platte an den Wirbelknochen mittels in die mit einer Senkung 47 versehenen Bohrungen 48 steckbaren Knochenschrauben

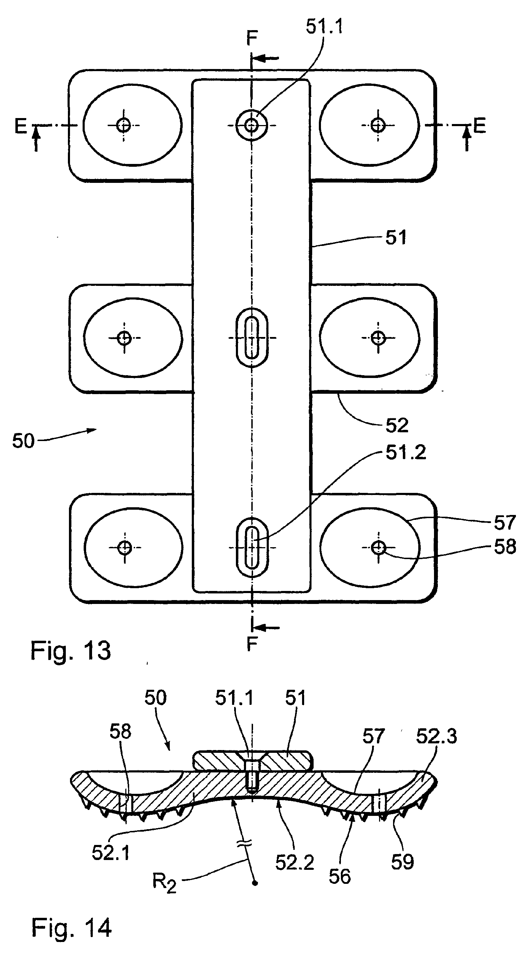

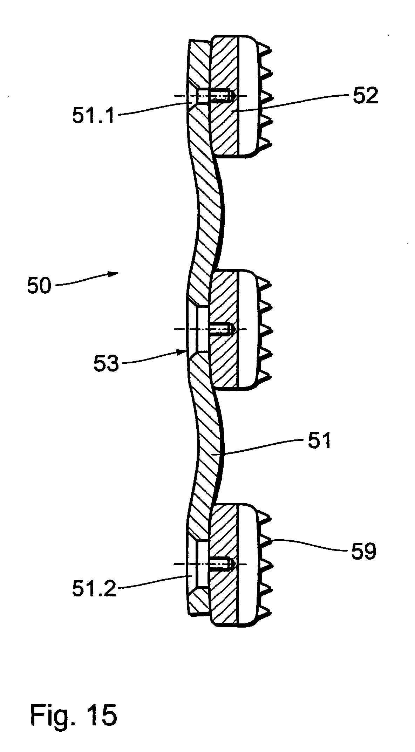

Die Figuren 13, 14 und 15 zeigen die Draufsicht und Schnittansichten einer Osteosynthese-Platte 50, die aus den Modulen 51 und 52 eines Modulsystems auf einfache Weise durch Verschrauben der einzelnen Systemelemente gebildet ist.

Als Systemelemente sind stabförmige Träger 51 bzw. als an den Trägern befestigbare, eine im wesentlichen gleiche Länge aufweisende Querstege 52 vorgesehen. Die Trägermodule 51 der Osteosynthese-Platte 50 sind in Längserstreckung wellenförmig ausgebildet, wobei die Querstege 52 an der Unterseite der Wellenmaxima 53 angeordnet sind.

Die Querstege 52 weisen im Mittelbereich 52.1 eine in Längsrichtung des Steges verlaufende konkave Wölbung 52.2 mit einem Radius R₂ auf, wobei an den Kontaktbereichen 52.3 der Querstege jeweils eine Ausformung vorgesehen ist, die eine bezüglich der konkaven Wölbung entgegengesetzt gerichtete, konvexe Gegenwölbung 56 bildet. Die Querstege 52 werden jeweils durch Verschrauben mit dem Träger 51 befestigt. Die dazu in den Träger eingearbeiten Bohrungen 51.1, 51.2 sind zumindest teilweise als Langlöcher 51.2 ausgebildet und ermöglichen eine Variation des relativen Abstand benachbart angeordneter Querstege 52. An den Endbereichen 52.3 der Querstege 52 sind weiterhin eine Senkung 57 aufweisende Bohrungen 58 vorgesehen, durch welche die zum Fixieren der Osteosynthese-Platte 50 an den Wirbelknochen erforderlichen (nicht dargestellten) Knochenschrauben geführt werden. Die relativ großflächige Senkung 57 weist die Form einer Kugelkappe auf und erleichtert dadurch das Plazieren der Knochenschrauben.

Die an der Unterseite der Querstege 52 angeordneten Dornen 59 unterstützen die Lagefixierung der osteosynthetischen Platte 50 beim Verschrauben mit den Wirbelknochen.

Durch den Einsatz von in der Länge unterschiedlichen Trägem 51 und Nutzung der hier vorgesehenen Langlöcher 51.2 sind mit den durch formgebendes Stanzen hergestellten Systemelementen 51, 52 des Modulsystems osteosynthetische Platten 50 bildbar, welche in ihren Abmessungen den anatomischen Bedingungen des jeweiligen Patienten ohne größere Schwierigkeiten angepaßt werden können.

Die Erfindung beschränkt sich in ihrer Ausführung nicht auf die vorstehend angegebenen bevorzugten Ausführungsbeispiele. Vielmehr ist eine Anzahl von Varianten möglich, welche von der dargestellten Lösung auch bei grundsätzlich anders gearteten Ausführungen Gebrauch macht.

## Patentansprüche

1. Zur Stabilisierung der relativen Stellung von Wirbelknochen, insbesondere von Wirbelknochen (2, 3) im Bereich der menschlichen Wirbelsäule, dienende Osteosynthese-Platte (1, 10, 20, 30, 40, 50), welche in Längs- und Querrichtung jeweils eine konkave Wölbung (4.1, 4.2, 14.2, 24.1, 24.2, 31.2, 42.2, 52.2) und in ihren mit den Wirbelknochen in Kontakt zu bringenden Bereichen (5, 15, 25, 31.3, 42.3, 52.3) zum Einbringen von Schraubmitteln angeordneten Bohrungen (8, 18, 28, 38, 48, 58) aufweist, dadurch gekennzeichnet, daß an den Kontaktbereichen der Platte jeweils eine Ausformung vorgesehen ist, die eine bezüglich der Wölbungen (4.1, 4.2, 14.2, 24.1, 24.2, 31.2, 42.2, 52.2) entgegengesetzt gerichtete, konvexe Gegenwölbung (6, 16, 26, 36, 46, 56) bildet.

2. Osteosynthese-Platte nach Anspruch 1, dadurch gekennzeichnet, daß der Radius der Wölbungen (4.1,4.2,14.2, 24.1, 24.2, 31.2, 42.2, 52.2) und der Radius der Gegenwölbung (6, 16, 26, 36, 46, 56) so gewählt ist, daß bei an den Wirbelknochen fixierter Platte zwischen Wirbelknochen und Platte ein Freiraum verbleibt.

3. Osteosynthese-Platte nach Anspruch 1 oder 2, dadurch gekennzeichnet, daß die Wölbungen (4.1, 4.2, 14.2, 24.1, 24.2, 6, 16, 26) bevorzugt eine Kugelkappe bilden.

4. Osteosynthetese-Platte nach Anspruch 3, dadurch gekennzeichent, daß der Radius (R₃) der Gegenwölbung (6, 16, 26, 36, 46, 56) einen Wert aufweist, welcher im wesentlichen der Größe des Radius (R₁) der Wölbung (4.1,24.1) in der Längsrichtung der Platte entspricht.

5. Osteosynthese-Platte nach einem der vorhergehenden Ansprüche, gekennzeichnet durch eine schmetterlingsförmige Ausbildung.

6. Osteosynthese-Platte nach einem der Ansprüche 1 bis 4, gekennzeichnet durch eine U-förmige oder Doppel-T-förmige Ausbildung.

7. Osteosynthese-Platte nach einem der Ansprüche 1 bis 4, gekennzeichnet durch eine Ausbildung als doppeltes Doppel-T.

8. Osteosynthese-Platte nach einem der vorhergehenden Ansprüche, dadurch gekennzeichnet, daß die Materialdicke der, bevorzugt durch formgebendes Stanzen, aus einem Blech gefertigten Platte (1, 10, 50) einen im wesentlichen konstanten Wert aufweist.

9. Osteosynthese-Platte nach einem der Ansprüche 1 bis 6, dadurch gekennzeichnet, daß die Kontaktbereiche der Platte (20), welche bevorzugt durch Schmieden, Gießen oder eine spangebende Bearbeitung hergestellt ist, eine im Verhältnis zum Mittelbereich größere Materialdicke aufweisen.

10. Osteosynthese-Platte nach einem der vorhergehenden Ansprüche, dadurch gekennzeichnet, daß an der proximalen Seite der die Gegenwölbung (6, 16, 56) aufweisenden Endbereiche Lagefixierungsmittel (9, 19, 59) vorgesehen sind.

11. Osteosynthese-Platte nach Anspruch 10, dadurch gekennzeichnet, daß die Lagefixierungsmittel (9, 19, 59) auf der Gegenwölbung (6, 16, 56) gleichmäßig verteilt angeordnet sind und sich im wesentlichen in Richtung der Achse der Bohrungen (8, 18, 58) erstrecken.

12. Osteosynthese-Platte nach Anspruch 10 oder 11, dadurch gekennzeichnet, daß die Lagefixierungsmittel (9, 19, 59) im wesentlichen dornen- oder noppenförmig ausgebildet sind.

13. Osteosynthetische Platte nach einem der Ansprüche 1 bis 9, dadurch gekennzeichnet, daß die an den Wirbelknochen (2, 3) anliegende Plattenseite zumindest im Bereich der Gegenwölbung (26, 36, 46) eine rauhe Oberfläche aufweist.

14. Osteosynthetische Platte nach Anspruch 13, dadurch gekennzeichnet, daß eine durch Plasmabeschichtung oder durch Sandstrahlen aufgerauhte Oberfläche vorgesehen ist.

15. Osteosynthese-Platte nach einem der vorhergehenden Ansprüche, dadurch gekennzeichnet, daß die an den Kontaktbereichen (5, 15, 25, 52.3) der Platte vorgesehenen Bohrungen (8, 18, 28, 38, 48, 58) eine Senkung (7, 17, 27, 37, 47, 57), bevorzugt eine sphärische Senkung (17, 57), aufweisen.

16. Osteosynthese-Platte nach Anspruch 15, dadurch gekennzeichnet, daß in den Bohrungen (38) ein, bevorzugt als sich radial nach innen erstreckende Haltenase ausgebildetes, Sicherungsmittel (34) vorgesehen ist, welches ein unbeabsichtigtes Herausfallen einer in der Bohrung befindlichen Knochenschraube verhindert.

17. Modulsystem zur Herstellung einer Osteosynthese-Platte nach Anspruch 7, gekennzeichnet durch stabförmige, in der Länge unterschiedliche Träger (51) und an den Trägern (51) befestigbare, eine im wesentlichen gleiche Länge aufweisende Querstege (52).

18. Modulsystem nach Anspruch 17, dadurch gekennzeichnet, daß die Träger (51) in Längserstreckung wellenförmig ausgebildet und die Querstege (52) an der Unterseite der Wellenmaxima (53) befestigbar sind.

19. Modulsystem nach Anspruch 17, dadurch gekennzeichnet, daß die Querstege (52) in Längsrichtung eine konkave Wölbung (52.2) und an ihren Kontaktbereichen jeweils eine Ausformung aufweisen, die eine bezüglich der konkaven Wölbung entgegengesetzt gerichtete, konvexe Gegenwölbung (56) bildet, wobei in den Endbereichen zum Einbringen von Schraubmitteln angeordneten Bohrungen (58) vorgesehen sind.

20. Modulsystem nach Anspruch 16, dadurch gekennzeichnet, daß die Querstege (52) jeweils durch Verschrauben mit dem Träger (51) verbindbar sind.
